(19) Europäisches Patentamt
European Patent Office
Office européen des brevets

(11) Publication number: 0 154 476
B1

(12) EUROPEAN PATENT SPECIFICATION

(45) Date of publication of patent specification: 16.05.90

(51) Int. Cl.$^5$: C 07 J 71/00, A 61 K 31/58

(21) Application number: 85301239.1

(22) Date of filing: 25.02.85

# (54) Steroid compound.

(30) Priority: 25.02.84 GB 8404980

(43) Date of publication of application:
11.09.85 Bulletin 85/37

(45) Publication of the grant of the patent:
16.05.90 Bulletin 90/20

(84) Designated Contracting States:
AT CH DE FR LI NL SE

(56) References cited:
GB-A-1 123 770
GB-A-2 010 278

CHEMICAL PHARMACEUTICAL BULLETIN, vol. 29, no. 9, September 1981, pages 2478-2484; Tokyo, JP. YUJI MORI et al.: "Synthesis and stereochemistry of the metabolites of 2alpha-cyano-4alpha, 5alpha-epoxy-17beta-hydroxyandrostan-3-one.".

(73) Proprietor: STERWIN AG.
Zeughausgasse 9
CH-6300 Zug (CH)

(72) Inventor: Jones, William Edward
16, Yewens
Chiddingfold Surrey GU8 4SD (GB)
Inventor: Andrews, Roderic Stafford
12, Thornlea Hepscott
Morpeth Northumberland NE61 6NY (GB)

(74) Representative: Green, Alan James et al
SANDERSON & CO. European Patent Attorneys
34, East Stockwell Street
Colchester Essex CO1 1ST (GB)

EP 0 154 476 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to a new pharmaceutically-active steriod compound and, in particular, to a metabolite of the compound having the common name "trilostane", which metabolite has significantly higher activity then the parent compound.

The compound "trilostane", which is 2α-cyano-4α,5α-epoxyandrostan-17β-ol-3-one having the formula:

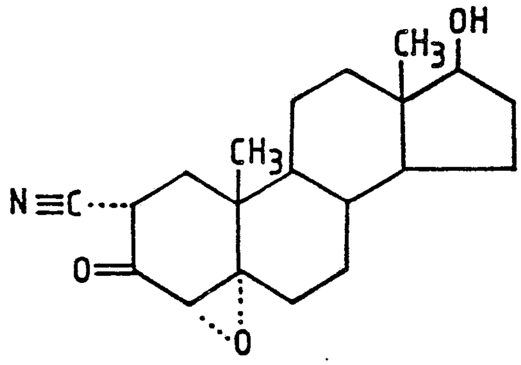

(I)

is described, along with related compounds in, for example, British Patent Specification No. 1,123,770 and U.S. Patent Specification No. 3,296,295. Those earlier specifications describe the adrenocortical inhibiting properties of trilostane and related compounds, which generally have a ring structure represented by the following formula:

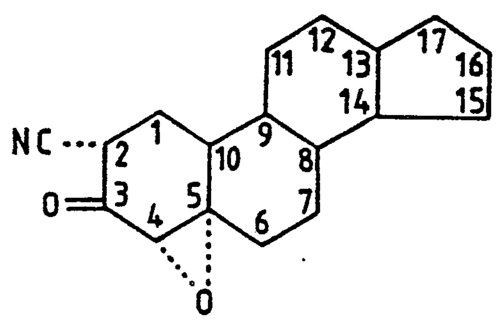

(II)

In the earlier disclosures, it is said that the exact nature of the steroid moiety is not critical, and it can be derived from any steroid of the general type known to exhibit hormonal or other pharmacological or endocrinological properties. Such steroid moieties are said to have from 19 to about 23 carbon atoms, not counting carbon content which may be provided by esterified hydroxy groups.

In particular, representative of the steroid moieties which are said to make up compounds of the earlier invention as disclosed in the British Specification are those having, at position 17, a hydroxy, acyloxy or oxo group or both a hydroxy and a lower alkyl or lower alkynyl group, as characteristic of the androgenic and anabolic steroids. The steroid moiety also is said to be one which can have one or more substituents at other positions of the nucleus, for example, hydroxy, ethyloxy or oxo groups at positions 6, 7, 11, 12, 14 (presumably "15" not "14" is intended) or 16; epoxy groups at adjacent positions, for example, at the 9,11-, 11,12- or 15,16- positions; halogen atoms, preferably fluorine, chlorine or bromine, for example, at the 6-, 7-, 9-, 12-, 16 or 17- positions; and lower-alkyl groups, for example, at the 6-, 7-, 11- or 16- positions. Furthermore, the steroid moiety is said to be one which can have one or more double bonds, for example, at the 6,7-, 9,11- or 16,17- positions, and the steroid moiety can also possess angular methyl groups at $C_{10}$ and $C_{13}$. Although in general terms the disclosure of the basic U.S. Specification is somewhat broader than that of the British Specification, for example, in that it is not limited to epoxy compunds, the same or similar considerations generally apply to the U.S. disclosure.

In published studies on the metabolism of trilostane, for example, by Mori et al in Chemical and Pharmaceutical Bulletin (Tokyo), 1981, *29* (9), pages 2478 to 2484 and pages 2646 to 2652, and by Suzuki et al in Proceedings of the 11th Symposium on Drug Metabolism and Action, November 6—7, 1979, pages S-10 and S-22, earlier workers have identified *inter alia* a metabole of trilostane in which in the above formula I the hydroxy group at the 17-position is replaced by a keto group. In the earlier published studies, the 17-keto metabolite is identified merely as one metabolite amongst a number of metabolites, and no information is given as to whether or not that or any other metabolite has any activity of itself.

While recognising that the 17-keto metabolite of trilostane is embraced within the generic disclosure of the above-mentioned earlier specifications, although it is not specifically disclosed therein, we believe that until now it has been thought that the metabolites of trilostane do not have the activity of the parent compound. Accordingly, it has previously been thought desirable to choose a form or means of administering trilostane which achieves the lowest possible ratio of 17-keto metabolite to parent compound, so that the patient receives maximum benefit from the active parent compound, and oxidation to the 17-keto metabolite and associated products is avoided for as long as possible.

Contrary to the above thinking, we have now found quite surprisingly that the 17-keto metabolite of trilostane has a relative *in vitro* potency compared with the parent compound of about 1.7 times as high as the parent compound on a weight for weight basis. Thus, we believe the 17-keto metabolite is the major active compound when trilostane is administered for clinical purposes, rather than the parent compound *per se*.

Accordingly, the present invention provides a compound of the formula:

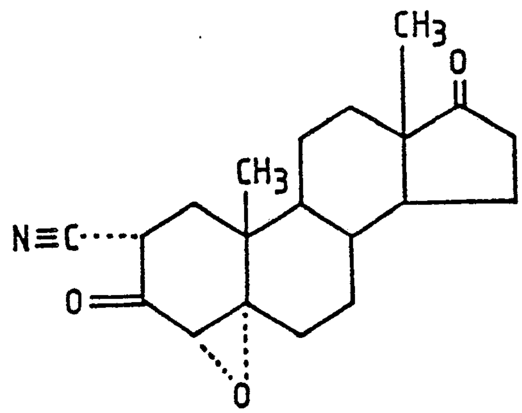

(III)

for use in a method of treatment of the human or animal body by therapy, in particular therapy to modulate or inhibit adrenal steroidogenesis, typically in any treatment requiring the administration of an adrenocortical inhibitor.

As will be appreciated by those familiar with the parent compound trilostane, the compund of the above formula III may be considered to exist in the 3-keto form shown and/or in the corresponding 3-enol form. It is to be understood, therefore, that wherever the keto form is shown or referred to herein and in the claims it is intended to embrace also the corresponding enol form or any mixture of the two forms.

The invention also provides a pharmaceutical composition, which composition comprises a compound of the above formula III, optionally together with one or more other active compounds, and including one or more pharmaceutically-acceptable excipients or carriers.

Such excipients or carriers may be solid, semi-solid or liquid as appropriate to the pharmaceutical form chosen, and may include a wide range of organic and inorganic solids and semi-solids, as well as aqueous and non-aqueous liquids. Of such materials, there may be used, for example, talc, gum arabic, starch, lactose, magnesium stearate or fatty substances of animal or vegetable origin such as cocoa butter, lanolin derivatives, paraffin derivatives or glycols. In addition, the excipient or carrier may be compounded with one or more wetting, dispersing or emulsifying agents and/or one or more preservatives as desired.

By way of example, the compositions of the invention may be presented as a tablet, a capsule, a granulate for suspension, a cream, an ointment, a suppository, a solution or a suspension. Preferably the composition of the invention includes a solid excipient or carrier with which the compound of formula III is mixed or co-granulated, for example, starch, lactose and/or magnesium stearate. More preferably, the composition is presented as a tablet or in an encapsulated form, and in the latter case the compound of formula III, together with any excipient or carrier is filled into the shell of the capsule.

Typically, the composition of the invention when in unit dosage form may comprise a unit dose of from about 30 to about 250 mg of the compound of formula III. The unit dose levels employed may be the same as or less than the corresponding unit dose levels for the parent compound, trilostane, depending on the regimen required compared with the parent compound. Thus, the compound of formula III may be administered at comparable dose levels to the parent compound, that is to say at a unit dose of from about 50 to about 250 mg, for example, about 60 mg, about 120 mg and about 240 mg, and more preferably from about 50 to about 120 mg, or at lower unit dose levels, for example, below about 100 mg.

As disclosed in our co-pending Application N. 83—29173, the parent compound trilostane is particularly effective when administered as a compound in particulate form consisting of particles having a mean equivalent sphere volume diameter of less than about 20 microns, at least 95% of the particles having a particle size of less than about 50 microns. The compound of the present invention also is preferably prepared and administered in the form having a reduced particle size, which more preferably is either one within the parameters laid down in the specification of the above-mentioned earlier Application with respect to particle size measured as a mean equivalent sphere volume diameter as generally defined

above, and in particular from about 5 to about 10 microns, or one within the additional or alternative particle size parameters expressed in terms of a specific surface area, preferably of at least about $2m^2g^{-1}$, more preferably from about 2 to about $5m^2g^{-1}$ e.g. from about 2 to about $4m^2g^{-1}$.

If desired, the active compound of the above formula III may be employed in admixture with one or more other active compounds such as trilostane *per se*.

As generally indicated above the compound of the invention can be used in the treatment of adrenal cortical hyperfunction as in hypercortisolism and primary aldosteronism. Thus, for example, it may be used in the treatment of Cushing's Syndrome and Conn's Syndrome, and also in the treatment of breast and prostate carcinoma.

The enhanced activity of the 17-keto metabolite of formula III above compared with trilostane itself can be seen from studies carried out as described below.

In the description which follows reference is made to the accompanying drawings in which:

Figure 1 is a graphical representation showing the relative inhibition of rat ovarian 3β-hydroxysteroid dehydrogenase activity by trilostane and the 17-keto trilostane metabolite of formula III, and

Figures 2(a) to 2(d) are graphical representations showing total plasma trilostane and 17-keto trilostane metabolite vs. trilostane-like bioactivity and plasma cortisol in four subjects studied.

In a first study, a cytochemical bioassay was employed to determine the relative potency of the 17-keto trilostane metabolite versus trilostane, in three experiments. From the results obtained, standard curves covering the range from 0.15 to 2.5 μM were prepared for the metabolite and the parent compound. Potency estimates were calculated by computer analysis of the relative slopes and errors around each dose point.

The cytochemical bioassay is based upon the reaction, within serial unfixed tissue sections of the dioestrous rat ovary, between the enzyme, 3β-hydroxysteroid dehydrogenase -$\Delta^5$-3-oxosteroid isomerase (3β HSD) contained therein, and added substrate, cofactors and a colourless tetrazolium salt. The reaction leads to the deposition of an intensely coloured blue insoluble formazan dye in the sections, and the amount of dye produced within a section over a given period (absorbance) is measured by means of a scanning integrating densitometer. If an inhibitor of 3β HSD, such as trilostane or 17-keto trilostane metabolite is added to the system, the amount of blue dye formed will be reduced. The more active the compound or the larger the amount added, the greater will be the reduction in absorbance.

The bioassay curves obtained are as shown in Figure 1 of the accompanying drawings, results being expressed as the mean $\pm$SD(n=4). As can be seen from Figure 1, both trilostane (upper curve — black circles) and the metabolite (lower curve — crosses) exhibit parallel dose response lines over the range of 0.15 to 2.5 μM. However, the fact that the curve for the metabolite is lower than that of trilostane indicates that the metabolite inhibits the enzyme to a greater extent than trilostane on a weight for weight basis, that is to say the metabolite is more active. In fact, as can be calculated from Figure 1, the metabolite is 1.71 times (95% confidence, 1.4—2.1) more active on a weight for weight basis than the parent molecule.

In the second study, trilostane as 2 × 60 mg capsules was taken orally by 4 healthy male volunteers between 0900 and 1000 hours each morning and the volunteers were fasted. A heparinised blood sample was taken before dosing and at various times up to 8 hours after dosing. Plasma was prepared and 2 to 5 ml aliquots were snap frozen and stored at −20°C.

Trilostane and its 17-keto metabolite were extracted from acidified plasma by a simple chloroform extraction procedure. In each case, one portion of the extract was taken for analysis by high pressure liquid chromatography (h.p.l.c.), and a second duplicate portion was taken for cytochemical bioassay as described above. Using those extracted portions, plasma trilostane and 17-keto metabolite concentrations were separately estimated by h.p.l.c. assay, and plasma trilostane-like bioactivity was measured by cytochemical bioassay. In addition, plasma cortisol was assayed by radioimmunoassay using the Amerlex Kit (obtained from Amersham International, Amersham, Buckinghamshire).

Since the bioactivity recorded relative to a standard curve for trilostane was substantially higher than the molar sum of circulating trilostane and 17-keto compound as assessed by h.p.l.c., the h.p.l.c. figures were corrected for a 1.71 times higher activity in the 17-keto compound, and the results plotted concentration vs. time as shown in Figures 2(a) to 2(d) of the accompanying drawings for each of the volunteer subjects. In Figures 2(a) to 2(d) the plots shown have the following key:

1. Black star positions — plasma cortisol (nmol/litre).
2. Black circle positions — molar sum of trilostane and 17-keto trilostane (times 1.71 to allow for increased potency) as determined by h.p.l.c.
3. Crosses — trilostane-like bioactivity as determined by cytochemical bioassay.

(The lower limit of detection of the cytochemical bioassay is $1 \times 10^{-6}$M: that for the h.p.l.c assay is $5 \times 10^{-7}$M. This is indicated by the horizontal dot/dash line below which are experimental points — triangles for h.p.l.c. and stars for cytochemical bioassay — where trilostane and 17-keto metabolite were undetectable).

As can be seen from Figures 2(a) to 2(d), the agreement between the bioassay of trilostane-like activity (crosses) and the molar sum of trilostane and 17-keto trilostane (times 1.71 to account for its increased potency) assayed by h.p.l.c. (black circles) is excellent.

In addition, a notable feature of the profiles shown is the clear relationship between the concentration of total active drug and cortisol in the plasma. Thus, where there are clear sustained peaks of trilostane and

17-keto metabolite, cortisol secretion is clearly shown to be suppressed and cortisol secretion recovers only when the drug levels decline.

The compound of the invention of the above formula III may be prepared by a variety of synthetic routes, in particular starting either from testosterone, that is a compound of the formula:

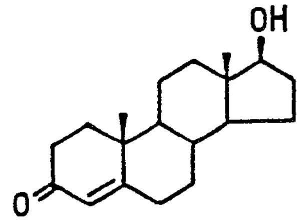

(IV)

or from androstenedione, that is a compound of the formula:

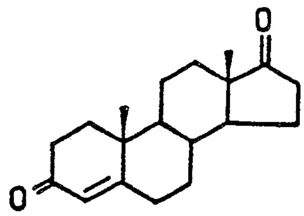

(V)

The preparative routes which are preferably followed can be summarised by the following reaction scheme:

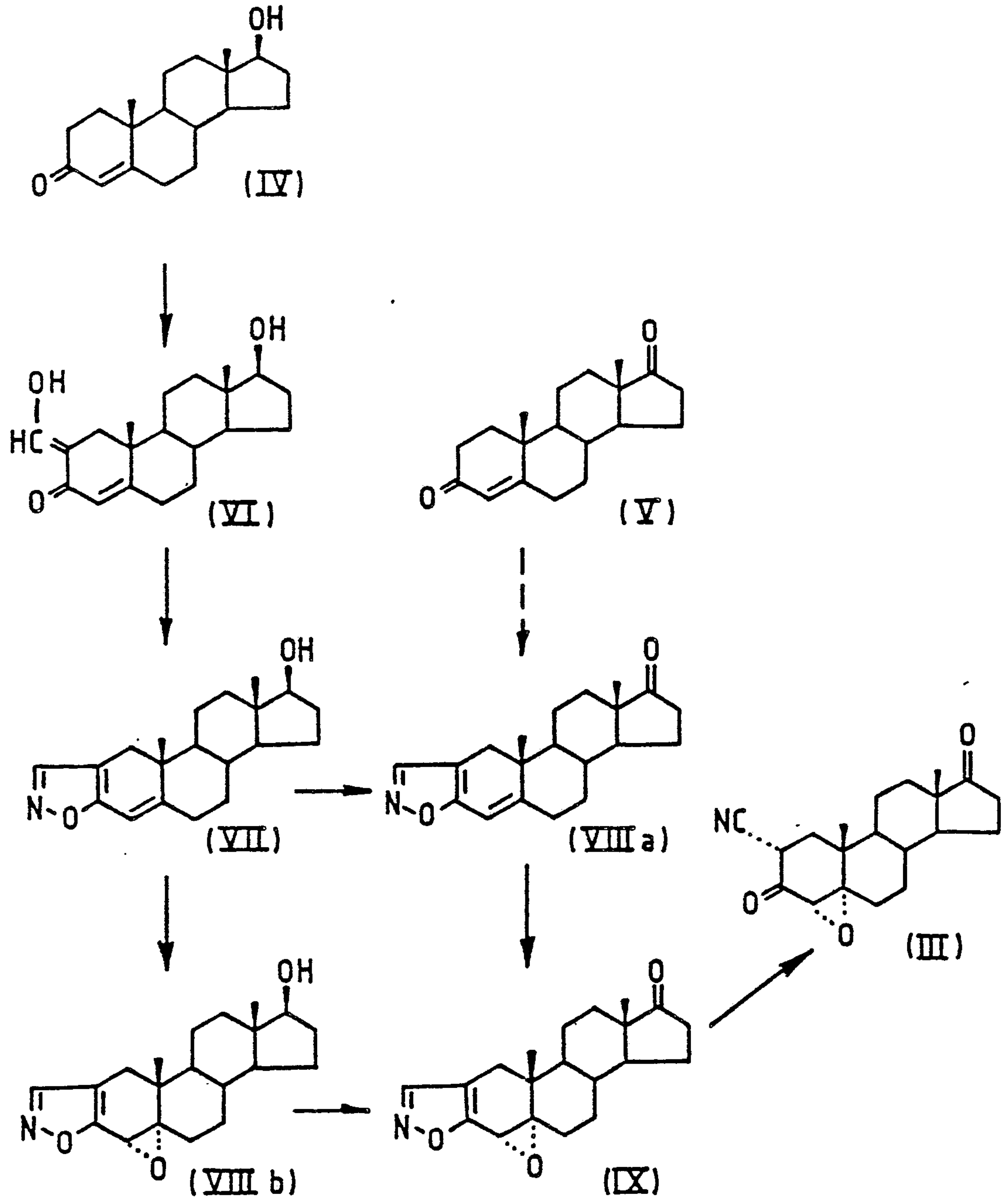

(IV) (VI) (V) (VII) (VIIIa) (III) (VIII b) (IX)

In accordance with the above reaction scheme the compound of the above formula III is preferably prepared by one of the following two preparative routes:

1. First Preparative Route:

17β-hydroxyandrost-4-eno[2,3-d]isoxazole of formula VII is oxidised to 17-ketoandrost-4-eno[2,3-d]isoxazole of formula VIII(a), which is then expoxidised to 17-keto-4α,5α-epoxyandrostano[2,3-d]isoxazole of formula IX, from which the compound of formula III is obtained by opening the isoxazole ring. For this route the starting material of formula VII may be prepared, for example, by reacting a 2-(1-hydroxy-alkylidene)-3-oxo compound of formula VI with hydroxylamine or an acid addition salt thereof — see, for example, U.S. Patent Specification No. 3,135,743. In turn, the compound of formula VI may be obtained from testosterone (compound IV).

Alternatively, the intermediate compound of formula VIII(a) may be obtained from androstenedione (compound V).

2. Second Preparative Route

17β-hydroxy-4α,5α-epoxyandrostano[2,3-d]isoxazole of formula VIII(b) is oxidised to 17-keto-4α,5α-epoxyandrostano[2,3-d]-isoxazole of formula IX, from which the compound of formula III is obtained by opening the isoxazole ring. Again the starting material of formula VIII(b) may be obtained from testosterone (compound IV) via the compound of formula VI, which is reacted with hydroxylamine to give the compound of formula VII, and which in turn is epoxidised to the starting material of formula VIII(b).

In either of the above preparative routes, a number of reagents capable of oxidising an alcohol to a carbonyl function may be employed in the appropriate oxidation step, for example:

Sodium dichromate,

Potassium dichromate e.g., using a phase transfer catalytic (PTC) reaction,

Potassium permanganate — under acidic, basic or netural conditions, or using a phase transfer catalyst,

Manganese dioxide,

Jones Reagent (chromium trioxide/sulphuric acid/water),

Dimethyl sulphoxide (using a Swern procedure),

Ceric ammonium nitrate,

Pyridinium dichromate,

Pyridinium chlorochromate,

Silver carbonate on Celite,

Lead tetraacetate in pyridine,

Iodosobenzene,

Acetone/aluminium isopropoxide (Merwein-Pondorff-Verley-Oppenauer oxidation reaction),

N-chlorosuccinimide,

N-bromosuccinimide,

Sodium persulphate,

Copper chromite, or

The trityl carbonium ion method.

Furthermore, opening of the steroido [2,3-d] isoxazole ring may be effected using a strong base, for example, postassium hydroxide. In that respect, while any strong base may be used, alkali metal hydroxides and alkoxides are preferred, more preferably with the reaction being carried out using an alkoxide in an anhydrous medium.

Also, epoxidation may be effected using any oxidising agent capable of converting an olefinic double bond to an epoxide. Thus, for example, hydrogen peroxide (under alkaline conditions), or a percarboxylic acid, e.g. perbenzoic, metachloroperbenzoic, monoperphthalic or peracetic acid, may be employed.

As can be seen from the specific Examples below, both of the above preparative routes, if necessary taken with the isolation steps described below, afford a product of high purity. Accordingly, the invention also includes a compound of the formula III in an isolated and essentially pure form, in particular a compound having a melting point of from about 230° to about 240°C, e.g. 235° to 238°C.

In addition, it is theoretically possible to employ the route used by Mori et al in their first paper cited above. In that route, trilostane of the above formula I is oxidised e.g. using an oxidising agent as described above, to 2-cyano-4α,5α-epoxyandrost-1-en-3,17-dione as follows:

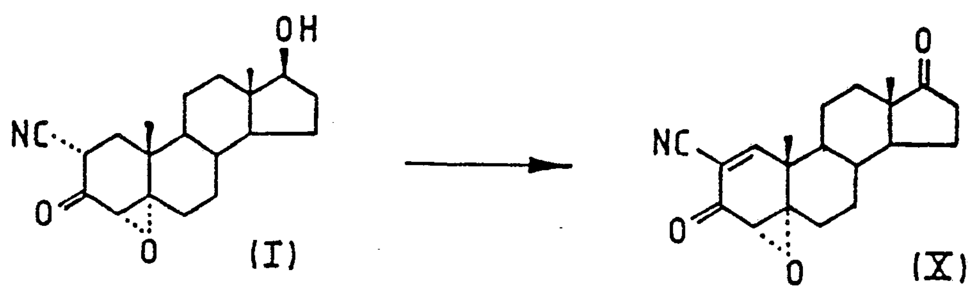

(I) (X)

The thus-prepared compound of formula X is then selectively reduced e.g. using hydrogen and a catalyst such as palladium on charcoal, to the desired compound of formula III.

It is to be noted, however, that the melting point for the isolated product quoted by Mori et al is far in excess of those reported in the specific Examples below. Accordingly, this route as specifically described by Mori et al may not lead to the compound of the invention and is therefore not a process in accordance with the present invention. However, it is to be understood that the invention includes processes for the preparation of a compound of the formula III as outlined above for the first and second preparative routes.

As well as the above-described steps, the processes of the invention may include one or more further steps of bringing the thus-prepared compound into the desired particle size form, for example, as described in co-pending Applications Nos. 83—30—6665.7 and 84—30—7432.9. Additionally or alternatively, the processes may include one or more further steps of blending the compound of the invention with one or more pharmaceutically-acceptable excipients or carriers, and optionally thereafter

bringing the thus-formed composition into a unit dosage form, for example, as a tablet, a capsule, a granulate for suspension, a cream, an ointment, a suppository or a suspension. In particular, the processes of the invention may include the additional steps of granulation and terminal blending subsequent to isolation of the compound of the invention, e.g. in a reduced particle size form.

The compound prepared in the desired form by one of the above routes may be isolated from the reaction mixture by, for example, filtration, then washed and dried. A variety of drying techniques may be employed which may include one or more of suction drying, tray drying and oven drying. In addition, as may be necessary, one or more of the various steps of the processes of the invention may be carried out under an inert atmosphere e.g. under an atmosphere of nitrogen.

The routes for the preparation of the compound of the invention, together with formulations containing the compound, are illustrated in the following specific Examples.

## Example 1

### A. First Preparative Route

a) 17-ketoandrost-4-eno[2,3-d]isoxazole [compound VIII(a)]

To a stirred suspension of 106 g of pyridinium chlorochromate in 850 ml of dichloromethane, cooled in an ice bath, was added a solution of 100 g of 17β-hydroxyandrost-4-eno[2,3-d]isoxazole (compound VII) over a period of 1.5 hours. The temperature of the reaction mixture was maintained at approximately 15°C. After the addition was complete, the reaction mixture was stirred for a further 2 hours, then the dichloromethane solution was decanted from the black tarry residue and the residue was extracted twice with hot dichloromethane. The dichloromethane extractions were combined and washed twice with 500 ml of 2 M hydrochloric acid. The organic layer was then slurried twice with 7 g of silica gel to give a pale green solution. The solution was evaporated to dryness and the solid residue was slurried with methanol, collected by filtration and the residue washed with a little methanol. A small sample was removed and recrystallised from dichloromethane to give a sample m.p. of 251 to 253°C. The remainder (65.5 g) was used directly in the following preparation.

b) 17-keto-4α,5α-epoxyandrostano[2,3-d]isoxazole (compound IX)

To a stirred solution of 65.5 g of 17-ketoandrost-4-eno[2,3-d]isoxazole [compound VIII(a)] in 650 ml of dichloromethane were added portions of 43.6 g of metachloroperbenzoic acid over a period of 20 minutes. The reaction mixture was cooled to keep the temperature below 20°C. When the addition was complete, the reaction mixture was stirred overnight at room temperature, then the solids were removed by filtration and the residue was washed with a little chloroform. The combined filtrates were washed with dilute sodium bisulphite until a negative starch-iodide test was obtained, after which the organic layer was washed twice with dilute sodium bicarbonate solution and finally with a saturated brine solution. The organic layer was next dried over anhydrous magnesium sulphate, evaporated to dryness, *in vacuo*, after which the solid residue was slurried, with ice cooling, in methanol. The solid was finally collected by filtration and dried under suction.

The filter cake was recrystallised from dimethylformamide, the crystals were collected by filtration under reduced pressure, washed with cold methanol, and then dried overnight at 50°C *in vacuo*. Yield 41 g m.p., 276°C (with decomposition).

c) 2α-cyano-4α,5α-peoxyandrostan-3,17-dione. (compound III)

To a stirred suspension of 41 g of 17-keto-4α,5α-epoxyandrostano-[2,3-d]isoxazole (compound IX) in 1 litre of tetrahydrofuran were added portions of 19.5 g of sodium methoxide, over a period of 10 minutes. The reaction mixture was stirred at room temperature for 22 hours. After this time water was added to the reaction mixture and most of the tetrahydrofuran was removed by evaporation under reduced pressure. More water was added to give a suspension of the sodium salt. The suspension was acidified by the slow addition of a 1:1 solution of hydrochloric acid in water. The almost white solid was collected by filtration under reduced pressure, and the solids were washed with water and dried *in vacuo* for 2 hours. The solid was recrystallised from ethanol and dried at 50°C *in vacuo* overnight. Yield 31 g, m.p. 236 to 238°C (with decomposition).

Microanalytical data

| | Calculated | Found |
|---|---|---|
| %C | 73.37 | 73.13 |
| %H | 7.70 | 7.84 |
| %N | 4.28 | 4.19 |

In addition, the 17-keto structure was confirmed by infra red and nuclear magnetic resonance spectral analysis.

Example 2

B. Second Preparative Route

a) 17-keto-4α,5α-epoxyandrostano[2,3-d]isoxazole (compound IX)

To a stirred suspension of 15.1 g of pyridinium chlorochromate in 500 ml of dichloromethane cooled to 2°C in an ice-water bath was added a solution of 15.4 g of 17β-hydroxy-4α,5α-epoxyandrostano[2,3-d]isoxazole [compound VIII(b)] in 250 ml of dichloromethane over a period of 50 minutes. The reaction was stirred at 2°C for a further 16 hours, when the suspended solids were removed by filtration under reduced pressure, and the filtrate was extracted twice with 2M hydrochloric acid. The organic layer was dried over anhydrous sodium sulphate and 20 g of silica gel were added to the dry organic layer. The excess solvent was removed under reduced pressure to give the solid reaction mixture supported on free flowing silica. The product was obtained from the silica by elution with a solvent which comprised 10ß ethyl acetate in dichloromethane and collection of fractions. The fractions which contained the desired product were combined and evaporated to dryness to give a white crystalline material. Yield 12.8 g, m.p. 233 to 234°C (with decomposition).

b) 2α-cyano-4α,5α-epoxyandrostan-3,17-dione (compound III)

To a stirred suspension of 12.8 g of 17-keto-4α,5α-epoxyandrostano[2,3-d]isoxazole (compound IX) in 400 ml methanol were added 200 ml of methanol in which had been dissolved 1.5 g of sodium metal. The reaction mixture was stirred overnight. The clear solution was evaporated to half its original volume under reduced pressure and 300 ml of water and 170 ml of 1 M hydrochloric acid were added and the resulting suspension stirred for 0.5 hours. The solids were collected by filtration and then air-dried. The 12.3 g of crude material obtained were purified by dry column chromatography using 300 g silica as a support. The material was eluted from the column using chloroform, methanol and acetic acid — 94:4:2 (v/v) — as eluant and fractions of the eluate were collected. The fractions which contained pure material were combined and eluant removed under reduced pressure. The residue was recrystallised from ethanol to give colourless needles. Yield 6.4 g, m.p. 230 to 233°C (with decomposition).

In addition, the 17-keto structure was confirmed by infra red spectral analysis.

Example 3

B. Second Preparative Route

a) 17-keto-4α,5α-epoxyandrostano[2,3-d]isoxazole (compound IX)

To a stirred suspension of pyridinium dichromate (236.25 g) in dichloromethane (5.625 litres) in a 20 litre flange top reactor was added a solution of 17β-hydroxy-4α,5α-epoxyandrostano[2,3-d]isoxazole [compound VIII(b)] (187.5 g) in dichloromethane (5 litres). A further portion of dichloromethane (625 ml) was added and the mixture stirred for 120 hours at room temperature. A thin layer chromatography (TLC) examination (see Note 1 below) of the reaction mixture at this time showed unreacted compound VIII(b) present. A further portion of pyridinium dichromate (30 g) was added and stirring continued overnight. A TLC examination at this point confirmed the absence of compound VIII(b). Alumina (261 g — Brockman grade 1) was added and stirring continued for a further 2 hours. The solids present in the reaction mixture were removed by filtration through a bed of Hyflo filter aid and the bed washed with dichloromethane (2 × 1 litres). The filtrates from the two batches were combined and reduced to about half volume (12 litres) under reduced pressure on a rotary evaporator at a bath temperature not exceeding 32°C.

The concentrated dichloromethane solution was divided into three 4 litre batches and each batch washed successively with 1 M hydrochloric acid solution (3 × 1600 ml), saturated sodium bicarbonate (3 × 1500 ml) and saturated brine (1000 ml). The washed organic layers were combined and dried over 500 g of Drierite (calcium sulphate). The Drierite was removed by filtration under suction, and washed with dichloromethane (2 × 750 ml). The filtrate and washes were combined and treated with activated charcoal (2 × 260 g), and then filtered through Hyflo filter aid with the filter aid being washed with dichloromethane (2 × 1000 ml). The filtrate and washes were combined and evaporated to dryness under reduced pressure.

The yield of compound IX was 339.6 g (91%).

Note 1: TLC was performed by acidification of an aliquot from the reaction mixture and extraction into ethyl acetate. Merck 60F 254 plates were employed and the solvent systems were as follows:

Chloroform:methanol:acetic acid 96:2:2 by volume — silica

Dichloromethane:methanol 97:3 by volume — silica

Chloroform:ethyl acetate 90:10 by volume — alumina

b) 2α-cyano-4α,5α-epoxyandrostan-3,17-dione (compound III)

To a stirred suspension of the thus-prepared compound IX (325.4 g) in tetrahydrofuran (THF — 12 litres) in a 20 litre flange top reactor was added a solution of potassium hydroxide (99.5 g) in THF:water (2.76 litres : 0.98 litres) and the resulting mixture stirred at room temperature for 41 hours.

A TLC examination at this point showed the reaction was incomplete. A further portion of THF (3 litres) was added and mixture stirred for a further 24 hours. TLC examination showed the reaction was still incomplete due to the insolubility of compound IX. To overcome this, methanol (2.5 litres) was added and the mixture stirred overnight. TLC examination then showed the reaction had gone to completion.

The suspension was slowly added with stirring to distilled water (40 litres), the flask was washed with

methanol (0.5 litres) and the washings added to the water. A clear solution was obtained. The pH was carefully adjusted to from 4.5 to 5.0 by slowly adding with stirring, 1 M hydrochloric acid (about 2.5 litres). The resultant suspension was stirred out for 4 hours, then the product collected by filtration under suction to give a first crop which was washed with water (15 litres). The addition of the water to the filtrate and cooling to 10°C gave a second crop which was collected by filtration under suction and washed with water (15 litres). The two crops were dried at 60°C under reduced pressure for 18 hours.

This yielded: crop 1 177.7 g; crop 2 114.1 g

The two crops were shown by TLC examination, infra red spectral data IR and melting point (236°C to 237°C with decomposition) to be pure compound III. The crops were combined to give 291.8 g (90%) of final product as a white crystalline powder, but since the product obtained was highly crystalline with a surface area below the preferred specification limit, it was reworked as described below.

c) Controlled Precipitation of Compound III

In order to bring the particle size of the thus-prepared compound III within the preferred specification of from about 2 to about 4$m^2g^{-1}$, the compound was further treated in accordance with the method of Application No. 84—30—7432.9 as follows:

Under nitrogen, dimethylformamide (DMF — 1.12 litres) at 60°C was added to a flat-top reactor charged with compound III (160 g) and the mixture stirred using a Buddeberg stirrer (200 rpm) until all solids had dissolved.

The solution was filtered hot, then cooled to 33°C. Deionised water (1.12 litres) was then added over 4 minutes using a peristaltic pump and the suspension stirred out for 1 hour at ambient temperature, with the stirring rate being readjusted to 200 rpm after 5 minutes since coagulation resulted in a decrease in the stirring rate. The resulting solids were collected by filtration under suction, then washed with 50% aqueous DMF (2 litres), followed by water (5.5 litres), after which they were dried under suction with the aid of a rubber dam for 2 hours. Drying was completed in vacuo after 66 hours at 700 mBar/65°C and 5 h/1000 mBar/65°C to yield 150.2 g (94%) of compound III as a white crystalline powder melting at 235.2° to 237°C (with decomposition). Surface area measurements (Strohlein) gave duplicate values of 2.79 $m^2g^{-1}$ and 2.73 $m^2g^{-1}$, mean 2.76 $m^2g^{-1}$.

FORMULATIONS:

Example 4

| Capsules: | Formulations | 60 mg | 120 mg |
|---|---|---|---|
| | Active compound III | 60 mg | 120 mg |
| | Lactose | 86 mg | 172 mg |
| | Maize starch | 86.65 mg | 173.3 mg |
| | Magnesium stearate | 2.35 mg | 4.7 mg |
| | | 235 mg | 470 mg |

Preparation

These formulations are prepared by either conventional dry blending techniques or more preferably conventional wet granulation techniques. Encapsulation is effected by means of conventional equipment.

Example 5

| Tablets: | Formulations | 60 mg | 120 mg | 240 mg |
|---|---|---|---|---|
| | Active compound III | 60 mg | 120 mg | 240 mg |
| | Lactose | 80 mg | 160 mg | 320 mg |
| | Maize starch | 85 mg | 170 mg | 340 mg |
| | Magnesium stearate | 2 mg | 4 mg | 8 mg |
| | | 227 mg | 454 mg | 980 mg |

Preparation

The powdered ingredients are granulated by means of conventional wet granulation techniques. The tablets are compressed using conventional compression equipment to tablet or caplet form.

Example 6

Suppositories:

| Formulations | 60 mg | 120 mg | 240 mg |
|---|---|---|---|
| Active compound III | 60 mg | 120 mg | 240 mg |
| Suppository base | qs | qs | qs |

Preparation

The suppositories are prepared from a suitable suppository base (e.g. Suppocire, Witepsol, Novata, etc.) using conventional poured melt techniques or cold compression.

Example 7

Ointment:

| Formulations | 1% | 2.5% | 5% |
|---|---|---|---|
| Active compound III | 1% | 2.5% | 5% |
| Ointment base | to 100% | to 100% | to 100% |

Preparation

The ointments are prepared by incorporating the active compound into a suitable pre-prepared ointment base (e.g. white soft paraffin).

Example 8

Cream:

| Formulation | 1% | 2.5% | 5% |
|---|---|---|---|
| Active compound III | 1% | 2.5% | 5% |
| Cream base | to 100% | to 100% | to 100% |

Preparation

The creams are prepared by incorporating the active compound into a suitable pre-prepared cream base (e.g. aqueous cream B.P.)

Example 9

Suspension:

| Formulations | 1.2% | 2.4% | 4.8% |
|---|---|---|---|
| Active compound III | 1.2% | 2.4% | 4.8% |
| Citric acid (anhydrous) BP | 0.237% | 0.237% | 0.237% |
| Sorbitol solution BPC | 20% | 20% | 20% |
| Cetomacrogol 1000 BPC | 0.09% | 0.09% | 0.09% |
| Sodium phosphate BP | 0.63% | 0.63% | 0.63% |
| Sodium carboxy-methyl cellulose | 0.20% | 0.20% | 0.20% |
| Veegum K | 1.00% | 1.00% | 1.00% |
| Flavour | qs | qs | qs |
| Preservative | qs | qs | qs |
| Purified water | to 100% | to 100% | to 100% |

Preparation

The suspensions are prepared by conventional manufacturing techniques having regard to the processing properties of the ingredients. Finally, each suspension is passed through a homogeniser.

Example 10

A parenteral formulation was prepared by using the following ingredients:

| Ingredients | %w/v |
|---|---|
| Active compound III | 0.50 |
| Sodium phosphate (B.P.) | 0.60 |
| Sodium chloride (Ph. Eur) | 0.66 |
| Potassium hydroxide | 0.11 |
| Water for injection — balance to | 100% |

**Claims for the Contracting States: CH DE FR LI NL SE**

1. A compound of the formula:

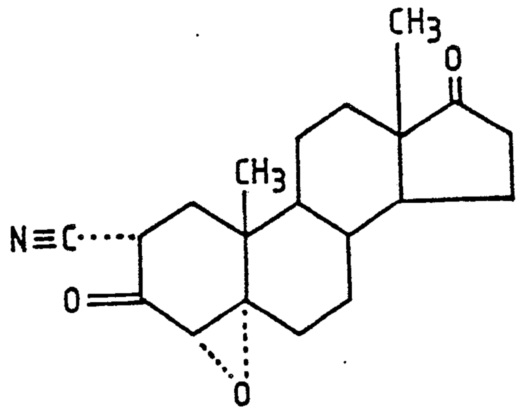

(III)

for use in a method of treatment of the human or animal body by therapy, in particular treatment of the human or animal body by therapy to modulate or inhibit adrenal steroidogenesis, and typically in therapy requiring the administration of an adrenocortical inhibitor.

2. A compound of the formula III defined in claim 1 in particulate form consisting of particles having a mean equivalent sphere volume diameter of less than 20 microns, preferably from 5 to 10 microns, at least 95% of the particles having a particle size of less than 50 microns.

3. A compound of the formula III defined in claim 1 in particulate form, the particle size being such as to provide a specific surface area of at least $2m^2g^{-1}$, preferably a specific surface area of from 2 to $5m^2g^{-1}$.

4. A compound of the formula III defined in claim 1 or a compound according to claim 2 or claim 3, which compound is in an isolated and essentially pure form, and preferably has a melting point of from 230° to 240°C.

5. A process for the preparation of a compound of the formula III defined in claim 1, which process comprises opening the isoxazole ring of a compound of the formula:

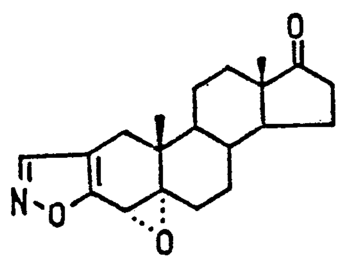

(IX)

preferably by treatment with a strong base, for example, an alkali metal hydroxide or alkoxide, and thereafter isolating said compound in a form for use in a method of treatment of the human or animal body by therapy, in particular treatment of the human or animal body by therapy to modulate or inhibit adrenal steroidogenesis, and typically in therapy requiring the administration of an adrenocortical inhibitor.

6. A process according to claim 5, wherein the compound of formula IX is obtained by epoxidation of a compound of the formula:

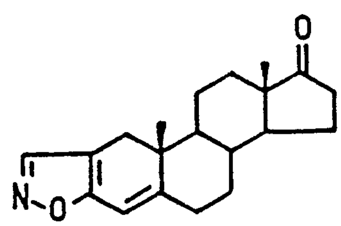

(VIII a)

the compound of formula VIII(a) preferably being obtained by oxidation of a compound of the formula:

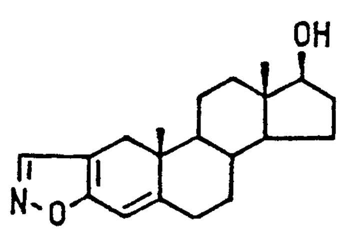

(VII)

7. A process according to claim 5, wherein the compound of formula IX is obtained by oxidation of a compound of the formula:

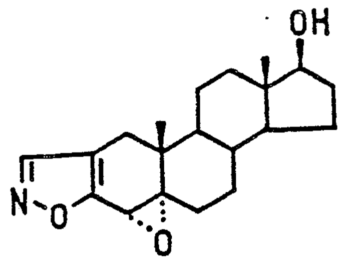

(VIII b)

the compound of formula VIII(b) preferably being obtained by epoxidation of a compound of the formula VII defined in claim 6.

8. A pharmaceutical composition, which composition comprises a compound according to any one of claims 2 to 4, and one or more pharmaceutically-acceptable excipients or carriers.

9. A pharmaceutical composition in unit dosage form, for example, in the form of a tablet or capsule, which composition comprises a compound of the formula III defined in claim 1 or according to any one of claims 2 to 4 in a unit dose of from 30 to 250 mg, and preferably from 50 to 120 mg, for example, below 100 mg.

10. A compound of the formula III defined in claim 1 when presented in a form for use in a method of treatment of the human or animal body by therapy, for example, as a package comprising a plurality of unit doses of a compound of the formula III and including instructions for use in a method of treatment of the human or animal body by therapy.

11. Use of a compound of the formula III as defined in claim 1 or according to any one of claims 2 to 4 for the manufacture of a medicament having enhanced activity for use in a method of treatment of the human or animal body by therapy to modulate or inhibit adrenal steroidogenesis.

**Claims for the Contracting State: AT**

1. A process for the preparation of a compound of the formula:

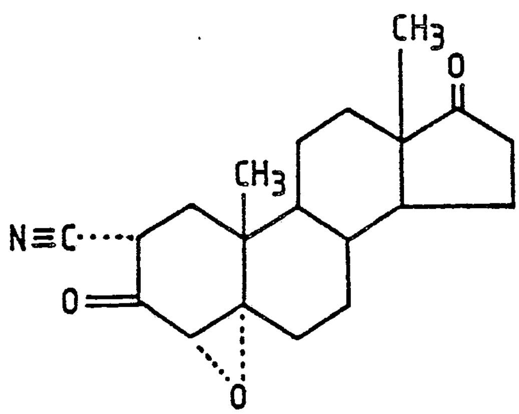

(III)

which process comprises opening the isoxazole ring of a compound of the formula:

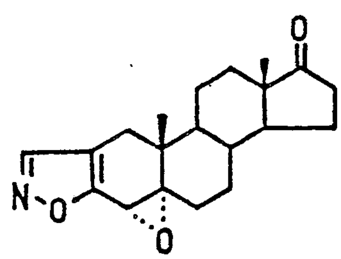

(IX)

preferably by treatment with a strong base, for example, an alkali metal hydroxide or alkoxide, and thereafter isolating said compound in a form for use in a method of treatment of the human or animal body by therapy, in particular treatment of the human or animal body by therapy to modulate or inhibit adrenal steroidogenesis, and typically in therapy requiring the administration of an adrenocortical inhibitor.

2. A process according to claim 1, wherein the compound of formula III is isolated in particulate form consisting of particles having a mean equivalent sphere volume diameter of less than 20 microns, preferably from 5 to 10 microns, at least 95% of the particles having a particle size of less than 50 microns.

3. A process according to claim 1, wherein the compound of formula III is isolated in particulate form, the particle size being such as to provide a specific surface area of at least $2m^2g^{-1}$, preferably a specific surface area of from 2 to $5m^2g^{-1}$.

4. A process according to any one of the preceding claims, wherein the compound of formula III is isolated in an essentially pure form, and preferably has a melting point of from 230° to 240°C.

5. A process according to any one of the preceding claims, wherein the compound of formula IX is obtained by epoxidation of a compound of the formula:

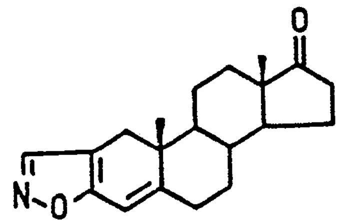

(VIIIa)

6. A process according to claim 5, wherein the compound of formula VIII(a) is obtained by oxidation of a compound of the formula:

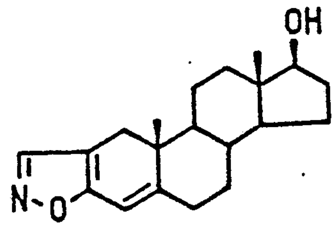

(VII)

7. A process according to any one of claims 1 to 4, wherein the compound of formula IX is obtained by oxidation of a compound of the formula:

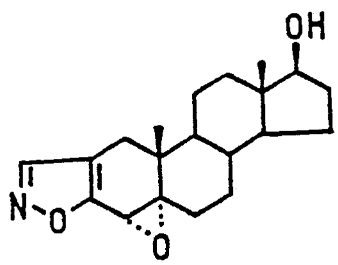

(VIII b)

8. A process according to claim 7, wherein the compound of formula VIII(b) is obtained by epoxidation of a compound of the formula VII defined in claim 6.

9. A process according to any one of the preceding claims, wherein the thus-prepared compound of formula III is mixed with one or more pharmaceutically-acceptable excipients or carriers to form a pharmaceutical composition.

**Revendications pour les Etats contractants: CH DE FR LI NL SE**

1. Composé du formule:

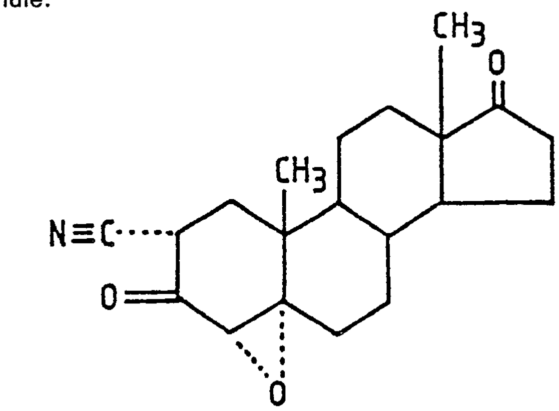

(III)

utilisable dans un procédé de traitement thérapeutique du corps humain ou animal, en particulier dans le traitement thérapeutique du corps humain ou animal pour moduler ou inhiber la stéroidogénèse de l'adrénaline, et de façon typique dans une thérapie nécessitant l'administration d'un inhibiteur adrénocortical.

2. Composé de formule III définie dans la revendication 1 sous forme particulaire consistant en des particules ayant un diamètre moyen de volume sphérique équivalent inférieur à 20 microns, de préférence de 5 à 10 microns, au moins 95% des particules ayant une taille inférieure à 50 microns.

3. Composé de formule III définie dans la revendication 1 sous forme particulaire, la taille des particules étant telle qu'elle donne une surface spécifique d'au moins 2 $m^2g^{-1}$, de préférence une surface spécifique de 2 à 5 $m^2g^{-1}$.

4. Composé de formule III définie dans la revendication 1 ou composé selon la revendication 2 ou la revendication 3, lequel composé est sous forme isolée et essentiellement pure, et a de préférence un point de fusion de 230° à 240°C.

5. Procédé de préparation d'un composé de formule III définie dans la revendication 1, lequel procédé comprend l'ouverture du cycle isoxazole d'un composé de formule:

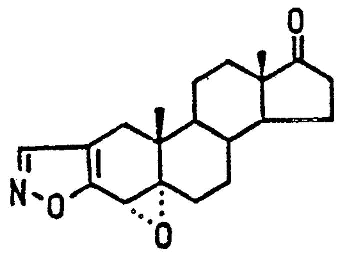

(IX)

de préférence par traitement avec une base forte, par exemple un hydroxyde ou alcoolate de métal alcalin, puis l'isolement dudit composé sous une forme utilisable dans un procédé de traitement thérapeutique du corps humain ou animal, en particulier de traitement thérapeutique du corps humain ou animal pour moduler ou inhiber la stéroidogénèse de l'adrénaline, et de façon typique dans une thérapie nécessitant l'administration d'un inhibiteur adrénocortical.

6. Procédé selon la revendication 5, dans lequel le composé de formule IX est obtenu par époxydation d'un composé de formule:

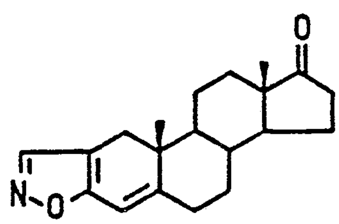

(VIII a)

le composé de formule VIII(a) étant de préférence obtenu par oxydation d'un composé de formule:

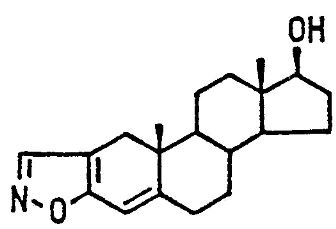

(VII)

7. Procéde selon la revendication 5, dans lequel le composé de formule IX est obtenu par oxydation d'un composé de formule:

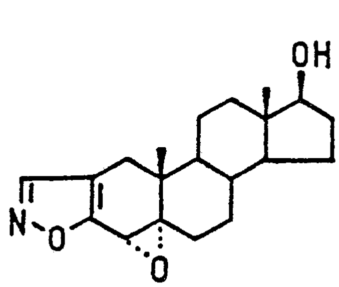

(VIII b)

le composé de formule VIII(b) étant de préférence obtenu par époxydation d'un composé de formule VII défini dans la revendication 6.

8. Composition pharmaceutique, laquelle composition comprend un composé selon l'une des revendications 2 à 4, et un ou plusieurs excipients ou véhicules acceptables du point de vue pharmaceutique.

9. Composition pharmaceutique sous forme de dose unitaire, par exemple sous forme d'un comprimè ou d'une capsule, laquelle composition comprend un composé de formule III définie dans la revendication 1 ou selon l'une des revendications 2 à 4 en une dose unitaire de 30 à 250 mg, et de préférence de 50 à 120 mg, par exemple inférieure à 100 mg.

10. Composé de formule III définie dans la revendication 1 qui se présente sous une forme utilisable dans un procédé de traitement thérapeutique du corps humain ou animal, par exemple sous forme d'un emballage comprenant une pluralité de doses unitaires d'une composé de formule III et comprenant un mode d'emploi, utilisable dans un procédé de traitement thérapeutique du corps humain ou animal.

11. Utilisation d'un composé de formule III telle que définie dans la revendication 1 ou selon l'une des revendications 2 à 4 pour la fabrication d'un médicament ayant une activité accrue utilisable dans un procédé de traitement thérapeutique du corps humain ou animal pour moduler ou inhiber la stéroidogénèse de l'adrénaline.

**Revendications pour l'Etat contractant: AT**

1. Procédé de préparation d'un composé de formule:

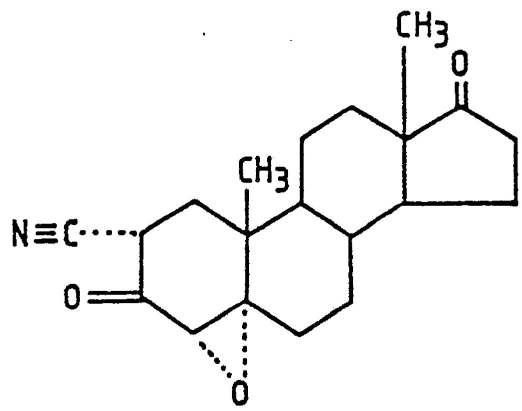

(III)

lequel procédé comprend l'ouverture du cycle isoxazole d'un composé de formule:

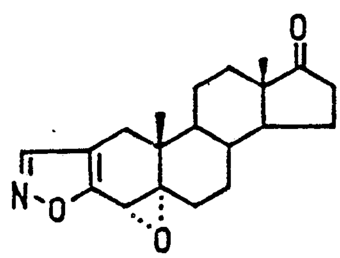

(IX)

de préférence par traitement avec une base forte, par exemple un hydroxyde ou alcoolate de métal alcalin, puis l'isolement dudit composé sous une forme utilisable dans un procédé de traitement thérapeutique du corps humain ou animal, en particulier de traitement thérapeutique du corps humain ou animal pour moduler ou inhiber la stéroidogénèse de l'adrénaline, et de façon typique dans une thérapie nécessitant l'administration d'un inhibiteur adrénocortical.

2. Procédé selon la revendication 1, dans lequel le composé de formule III est isolé sous forme particulaire consistant en des particules ayant un diamètre moyen de volume sphérique équivalent inférieur à 20 microns, de préférence de 5 à 10 microns, au moins 95% des particules ayant une taille inférieure à 50 microns.

3. Procédé selon la revendication 1, dans lequel le composé de formule III est isolé sous forme particulaire, la taille des particules étant telle qu'elle donne une surface spécifique d'au moins 2 $m^2g^{-1}$, de préférence une surface spécifique de 2 à 5 $m^2g^{-1}$.

4. Procédé selon l'une des revendications précédentes, dans lequel le composé de formule III est isolé sous une forme essentiellement pure, et a de préférence un point de fusion de 230° à 240°C.

5. Procédé selon l'une des revendications précédentes, dans lequel le composé de formule IX est obtenu par époxydation d'une composé de formule:

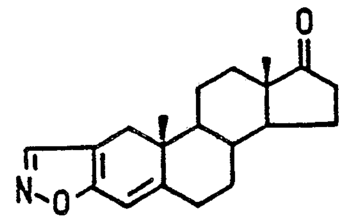

(VIII a)

6. Procédé selon la revendication 5, dans lequel le composé de formule VIII (a) est obtenu par oxydation d'un composé de formule:

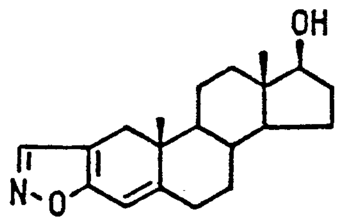

(VII)

7. Procédé selon l'une des revendications 1 à 4, dans lequel le composé de formule IX est obtenu par oxydation d'un composé de formule:

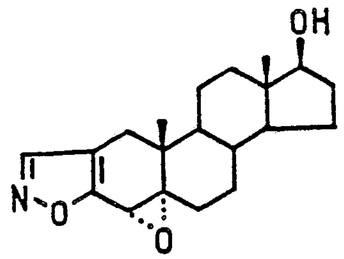

(VIII b)

8. Procédé selon la revendication 7, dans lequel le composé de formule VIII (b) est obtenu par époxydation d'un composé de formule VII définie dans la revendication 6.

9. Procédé selon l'une des revendications précédentes, dans lequel le composé de formule III ainsi préparé est mélangé avec un ou plusieurs excipients ou véhicules acceptables du point de vue pharmaceutique pour former une composition pharmaceutique.

**Patentansprüche für die Vertragsstaaten: CH DE FR LI NL SE**

1. Eine Verbindung der Formel:

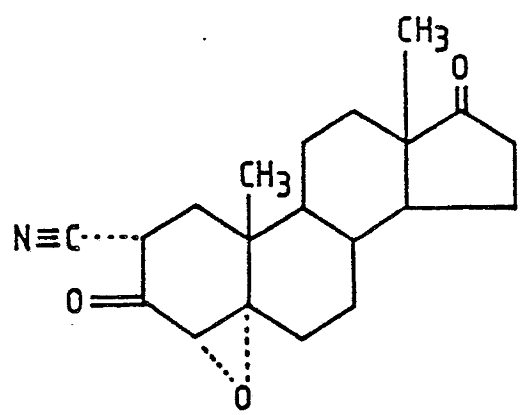

(III)

zur Verwendung in einem therapeutischen Verfahren zur Behandlung von menschlichen oder tierischen Körpern, insbesondere zur therapeutischen Behandlung des menschlichen oder tierischen Körpers, um adrenale Steroidgenese zu modulieren oder zu inhibieren und im typischen Falle in Therapien, die die Kontrolle eines adrenocortikalen Inhibitors erforderlich machen.

2. Eine Verbindung der Formel III nach Anspruch 1 in Partikelform, welche aus Partikeln mitt einem mittleren, äquivalenten Kugelvolumenquerschnitt von weniger als 20 µm, vorzugsweise von 5 bis 10 µm, besteht, wobei zumindest 95% der Partikel eine Partikelgröße von weniger als 50 µm aufweisen.

3. Eine Verbindung der Formel III nach Anspruch 1 in Partikelform, deren Partikel in einer solchen Größe vorliegen, daß eine spezifische Oberfläche von zumindest 2 $m^2g^{-1}$, vorzugsweise eine spezifische Oberfläche von 2 bis 5 $m^2g^{-1}$, erhalten wird.

4. Eine Verbindung der Formel III nach Anspruch 1 oder eine Verbindung nach den Ansprüchen 2 oder 3, welche in isolierter und prinzipiell reiner Form vorliegt und vorzugsweise einen Schmelzpunkt von 230° bis 240°C aufweist.

5. Ein Herstellungsverfahren zur Gewinnung einer Verbinding der Formel III nach Anspruch 1, bei dem die Öffnung des Isoxazol-Ringes einer Verbindung der Formel:

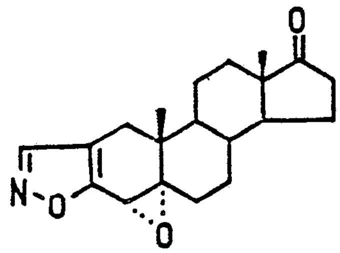

(IX)

vorzugsweise durch Behandlung mit einer starken Base, z.b. mit einem Alkalimetall-Hydroxid oder Alkoxid erfolgt, und nachfolgend die Verbindung in einer für ein therapeutisches Verfahren zur Behandlung von menschlichen oder tierischen Körpern verwendbaren Form isoliert wird, insbesondere zur therapeutischen Behandlung des menschlichen oder tierischen Körpers, um die adrenale Steroidgenese zu modulieren oder zu inhibieren, und im typischen Falle in Therapien, die die Kontrolle eines adrenocortikalen Inhibitors erforderlich machen.

6. Ein Herstellungsverfahren nach Anspruch 5, dadurch gekennzeichnet, daß die Verbindung der Formel IX durch Epoxidation einer Verbindung der Formel:

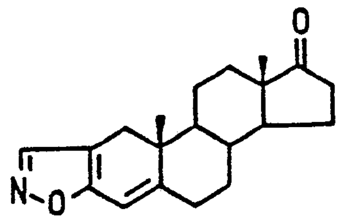

(VIII a)

erhalten wird, wobei die Verbindung der Formel VIII(a) vorzugsweise durch Oxidation einer Verbindung der Formel:

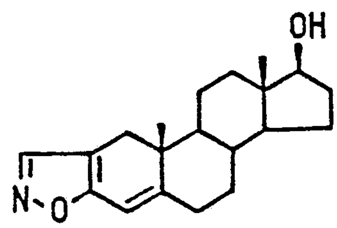

(VII)

erhalten wird.

7. Ein Herstellungsverfahren nach Anspruch 5, dadurch gekennzeichnet, daß die Verbindung der Formel IX durch Oxidation einer Verbindung der Formel:

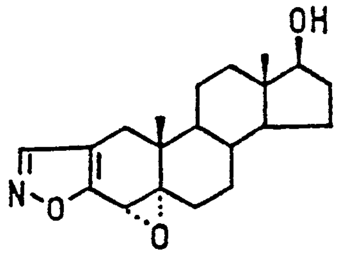

(VIII b)

erhalten wird, wobei die Verbindung der Formel VIII(b) vorzugsweise durch Epoxidation einer Verbindung der Formel VII nach Anspruch 6 erhalten wird.

8. Eine pharmazeutische Zusammensetzung, die eine Verbindung nach einem der Ansprüche 2 bis 4 und einen oder mehrere pharmazeutisch verträgliche Hilfs- oder Trägerstoffe enthält.

9. Eine pharmazeutische Zusammensetzung in Form einer Dosierungseinheit, beispielsweise in der Form einer Tablette oder Kapsel, die eine Verbindung der Formel III nach Anspruch 1 oder nach einem der Ansprüche 2 bis 4 in einer Dosierungseinheit von 30 bis 250 mg, vorzugsweise von 50 bis 120 mg, beispielsweise unter 100 mg, enthält.

10. Eine Verbindung der Formel III nach Anspruch 1 als in einer zur Verwendung in einem therapeutischen Behandlungsverfahren vorgelegten Form, z.B. als Packung, die mehrere Dosierungseinheiten einer Verbindung der Formel III, sowie Anweisungen zur Verwendung in einem therapeutischen Verfahren zur Behandlung von menschlichen oder tierischen Körpern enthält.

11. Verwendung einer Verbindung mit der Formel III nach Anspruch 1 oder nach einem der Ansprüche 2 bis 4 zur Herstellung eines Medikaments mit verstärkter Aktivität zur Verwendung in einem therapeutischen Verfahren zur Behandlung von menschlichen oder tierischen Körpern, um adrenale Steroidgenese zu modulieren oder zu inhibieren.

**Patentansprüche für den Vertragsstaat: AT**

1. Ein Herstellungsverfahren zur Gewinnung einer Verbindung der Formel:

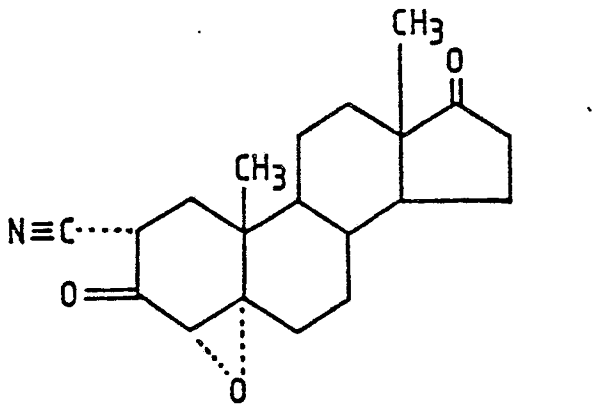

(III)

bei dem die Öffnung des Isoxazol-Ringes einer Verbindung der Formel:

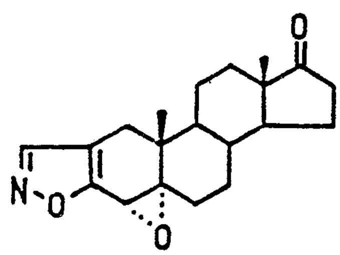

(IX)

vorzugsweise durch Behandlung mit einer starken Base, z.B. mit einem Alkalimetall-Hydroxid oder Alkoxid erfolgt, und nachfolgend die Verbindung in einer für ein therapeutisches Verfahren zur Behandlung von menschlichen oder tierischen Körpern verwendbaren Form isoliert wird, insbesondere zur therapeutischen Behandlung des menschlichen oder tierischen Körpers, um die adrenale Steroidgenese zu modulieren oder zu inhibieren, und im typischen Falle in Therapien, die die Kontrolle eines adrenocortikalen Inhibitors erforderlich machen.

2. Ein Herstellungsverfahren nach Anspruch 1, dadurch gekennzeichnet, daß die Verbindung der Formel III in Partikelform isoliert wird und aus Partikeln mit einem mittleren, äquivalenten Kugelvolumenquerschnitt von weniger als 20 µm, vorzugsweise von 5 bis 10 µm, besteht, wobei zumindest 95% der Partikel eine Partikelgröße von weniger als 50 µm aufweisen.

3. Ein Herstellungsverfahren nach Anspruch 1, dadurch gekennzeichnet, daß die Verbindung der Formel III in Partikelform isoliert wird, wobei die Partikel in einer solchen Größe vorliegen, daß eine spezifische Oberfläche von zumindest 2 $m^2g^{-1}$, vorzugsweise eine spezifische Oberfläche von 2 bis 5 $m^2g^{-1}$, erhalten wird.

4. Ein Herstellungsverfahren nach einem der vorgenannten Ansprüche, dadurch gekennzeichnet, daß die Verbindung der Formel II in prinzipiell reiner Form isoliert wird und vorzugsweise einen Schmelzpunkt von 230° bis 240°C aufweist.

5. Ein Herstellungsverfahren nach einem der vorgenannten Ansprüche, dadurch gekennzeichnet, daß die Verbindung der Formel IX durch Epoxidation einer Verbindung der Formel:

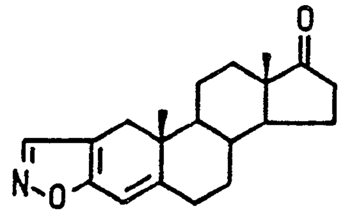

(VIII a)

erhalten wird.

6. Ein Herstellungsverfahren nach Anspruch 5, dadurch gekennzeichnet, daß die Verbindung der Form VIII (a) durch Oxidation einer Verbindung der Formel:

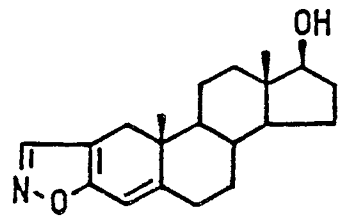

(VII)

erhalten wird.

7. Ein Herstellungsverfahren nach einem der Ansprüche 1 bis 4, dadurch gekennzeichnet, daß die Verbindung der Formel IX durch Oxidation einer Verbindung der Formel:

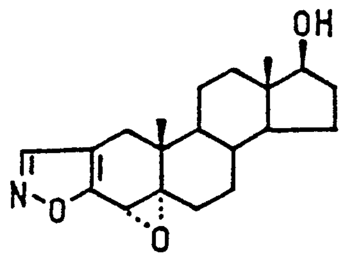

(VIII b)

erhalten wird.

8. Ein Herstellungsverfahren nach Anspruch 7, dadurch gekennzeichnet, daß die Verbindung der Formel VIII (b) durch Epoxidation einer Verbindung der Formel VII nach Anspruch 6 erhalten wird.

9. Ein Herstellungsverfahren nach einem der vorgenannten Ansprüche, dadurch gekennzeichnet, daß die auf diese Weise hergestellte Verbindung der Formel III mit einem oder mehreren pharmazeutisch verträglichen Hilfs- oder Trägerstoffen vermischt wird, um eine pharmazeutische Zusammensetzung zu erhalten.

FIG. 1

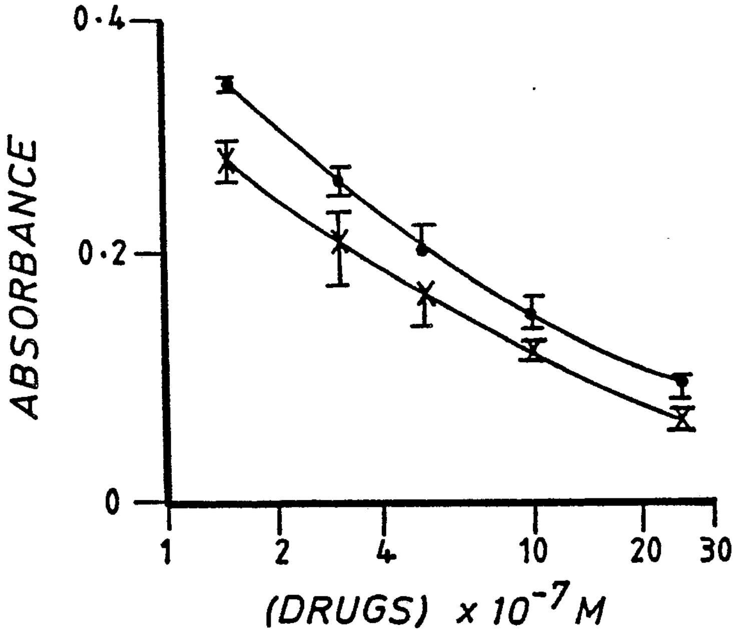

FIG.(2 a)

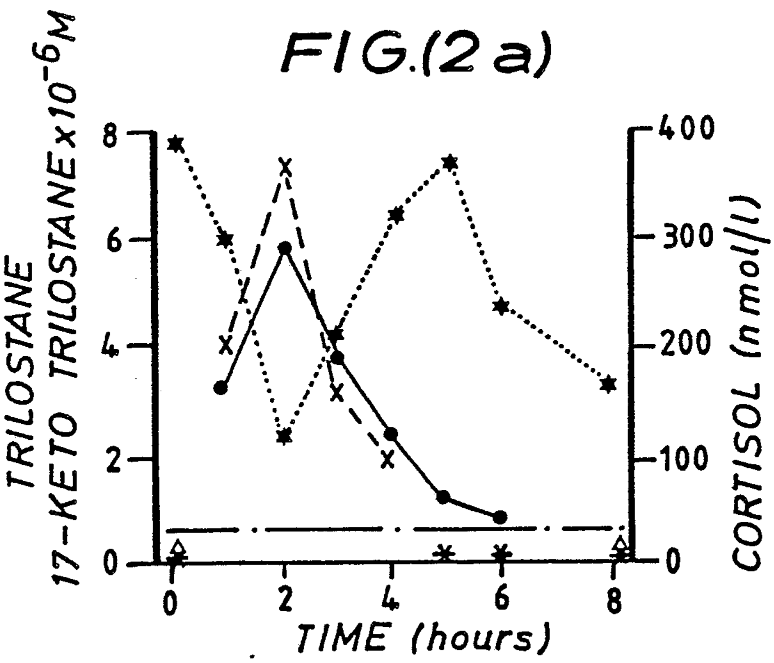
TRILOSTANE
17-KETO TRILOSTANE x10^-6 M
0
2
4
6
8
CORTISOL (nmol/l)
0
100
200
300
400
0
2
4
6
8
TIME (hours)

FIG. 2 (b)

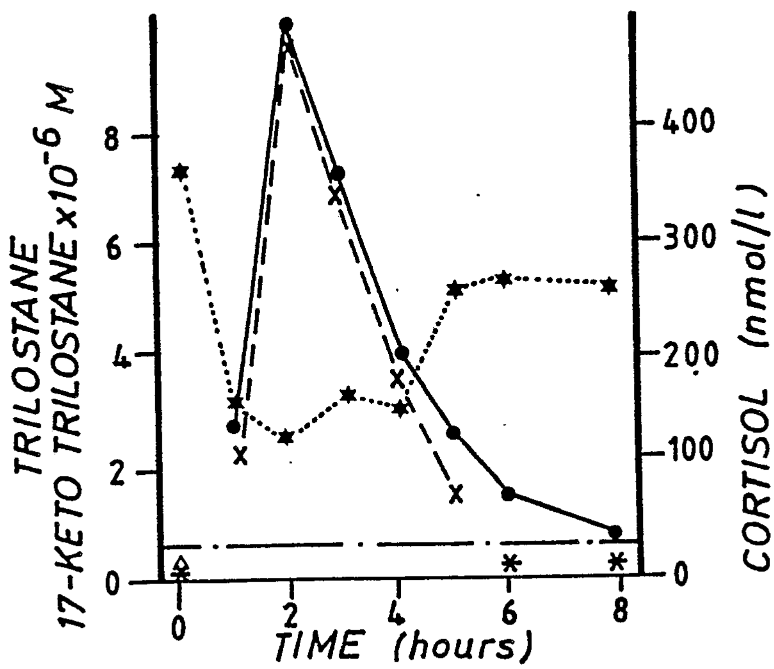
TRILOSTANE
17-KETO TRILOSTANE x10^-6 M
0
2
4
6
8
CORTISOL (nmol/l)
0
100
200
300
400
0
2
4
6
8
TIME (hours)

FIG. 2(c)

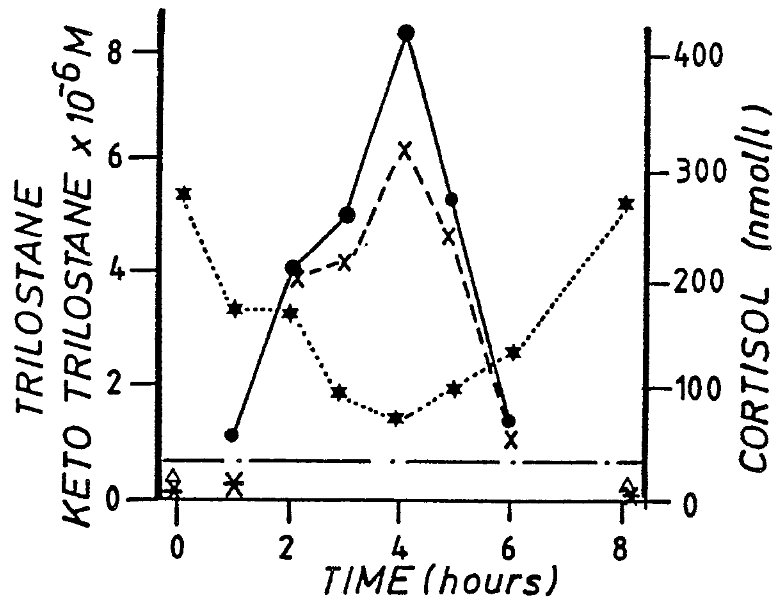
TRILOSTANE
KETO TRILOSTANE x 10⁻⁶ M
0
2
4
6
8
CORTISOL (nmol/l)
0
100
200
300
400
TIME (hours)

FIG. 2(d)

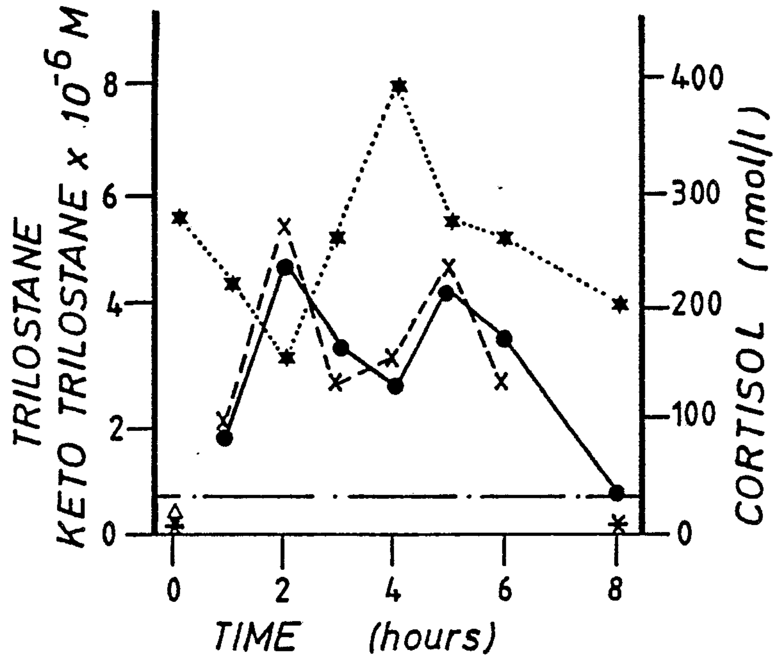
TRILOSTANE
KETO TRILOSTANE x 10⁻⁶ M
0
2
4
6
8
CORTISOL (nmol/l)
0
100
200
300
400
TIME (hours)